# EUROPEAN PATENT SPECIFICATION

(11) **EP 2 491 863 B1**
(45) Date of publication and mention of the grant of the patent: **19.06.2013**
(21) Application number: 12156224.3
(22) Date of filing: 20.02.2012
(51) Int. Cl.: A61B 6/00, A61B 6/02

(54) **Apparatus for tomosynthesis and mammography**
Vorrichtung zur Tomosynthese und Mammographie
Appareil de tomosynthèse et de mammographie

(30) Priority: 25.02.2011 IT BO20110085
(43) Date of publication of application: 29.08.2012
(73) Proprietor: I.M.S. Internazionale Medico Scientifica S.r.l., 40044 Pontecchio Marconi (Bologna) (IT)
(72) Inventor: Albanese, Achille, 40044 PONTECCHIO MARCONI (Bologna) (IT); Toniolo, Bruno, 40044 PONTECCHIO MARCONI (Bologna) (IT); Vecchio, Sara, 40044 PONTECCHIO MARCONI (Bologna) (IT); Vignoli, Paolo, 40044 PONTECCHIO MARCONI (Bologna) (IT); Borasi, Giovanni, 40044 PONTECCHIO MARCONI (Bologna) (IT)
(74) Representative: Bianciardi, Ezio

(56) References cited:
- DE-A1-102008 004 473
- US-A1- 2009 323 893
- US-A1- 2010 091 940

## Description

This invention relates to an apparatus for performing tomosynthesis (in particular, digital breast tomosynthesis) and mammography.

Known in the prior art are apparatuses designed to perform both digital breast tomosynthesis (DBT) and mammography.

These apparatuses comprise a source configured to emit X-rays and a detector configured to receive the X-rays emitted by the source.

It should be noted that the patient's breast being analysed is interposed between the X-ray source and the detector in such a way that the X-rays pass through it.

Generally speaking, the breast is placed on a suitable rest and compressed by a plate known as compressor.

As is known, in the breast being analysed X-rays are absorbed to a different extent by parts affected by tumour growths or the like as compared to parts without tumour growths. A radiographic image obtained from the X-rays received by the detector can thus be analysed to identify suspected tumours.

To use the apparatus for performing mammography, the X-ray source, the detector and the patient's breast must be positioned in a predetermined space relationship with each other. Processing of the X-rays received by the detector makes it possible to obtain a two-dimensional image of the patient's breast.

To perform digital breast tomosynthesis, on the other hand, the X-ray source is movable to allow the X-rays to be generated from a plurality of angles relative to the patient's breast.

One advantage of these apparatuses is the possibility of combining tomosynthesis with mammography in order to obtain a very effective and accurate diagnosis based on the combination of both techniques.

A need which is strongly felt by operators in this field is that for an apparatus for performing both tomosynthesis and mammography at a high speed while at the same time guaranteeing high quality X-ray images during both mammography and tomosynthesis. This invention therefore has for an aim to satisfy the aforementioned need by providing an apparatus for performing mammography and tomosynthesis which allows mammography / tomosynthesis to be performed at high speed while at the same time allowing high-quality images to be obtained.

Another aim of the invention is to provide an apparatus for performing mammography and tomosynthesis which makes it possible to locate quickly and accurately the trajectory of a needle through the patient's body in order to take a biopsy of biological tissue from a given part of the patient's body.

Yet another aim of the invention is to provide an apparatus for performing mammography and tomosynthesis in which the diffuse or secondary radiation received by the detector is reduced.

In accordance with the invention, this aim is achieved by an apparatus for performing mammography and tomosynthesis comprising the technical features set out in one or more of the appended claims.

The technical features of the invention according to the above mentioned aims are clearly described in the claims below and its advantages are more apparent from the detailed description which follows, with reference to the accompanying drawings which illustrate a non-limiting example embodiment of the invention and in which:
- Figures 1 and 2 are schematic front views of a first embodiment of the apparatus according to the invention in two respective configurations;
- Figure 1A illustrates another view of the apparatus according to the invention of Figure 1 where some parts have been cut away in order to better illustrate others;
- Figure 3 is a schematic front view of a second embodiment of the apparatus according to this invention;
- Figure 4 is a schematic front view of the first embodiment of Figures 1 and 2 equipped with a biopsy device;
- Figure 5 is a schematic front view of the second embodiment of the apparatus of Figure 3 equipped with a biopsy device;
- Figure 6 is a schematic side view of a detail of the apparatus of Figures 1-5 according to the viewing direction J1;
- Figure 7 is a schematic front view of a detail of the apparatus of Figures 1-5;
- Figure 8 shows a graph representing the flow of energy per unit area of an X-ray tube at a defined distance therefrom as a function of the angle to the central direction of emission in a plane at a right angle to the anode-cathode direction.

With reference to the accompanying drawings, the numeral 1 denotes an apparatus for tomosynthesis and mammography according to this invention.

More specifically, it should be noted that the apparatus 1 according to the invention allows both mammography and tomosynthesis to be performed on a breast M of a patient.

Hereinafter, the term "patient" is used to mean a person, whether male or female, subjected to analysis using the apparatus 1.

The apparatus 1 comprises an X-ray detector device 2 designed to receive and detect X-rays in a first, detection plane 3 (hereinafter also referred to as detection plane 3).

It should be noted that the detector device 2 is a substantially two-dimensional detector.

The detector device 2 comprises a sensor designed to detect a flow of X-rays incident upon the detection plane 3.

It should be noted that preferably, but without limiting the invention, the sensor of the detector device 2 is a solid state sensor based on amorphous selenium.

The apparatus 1 comprises a plurality of X-ray sources 4.

Preferably, the X-ray sources 4 are X-ray tubes.

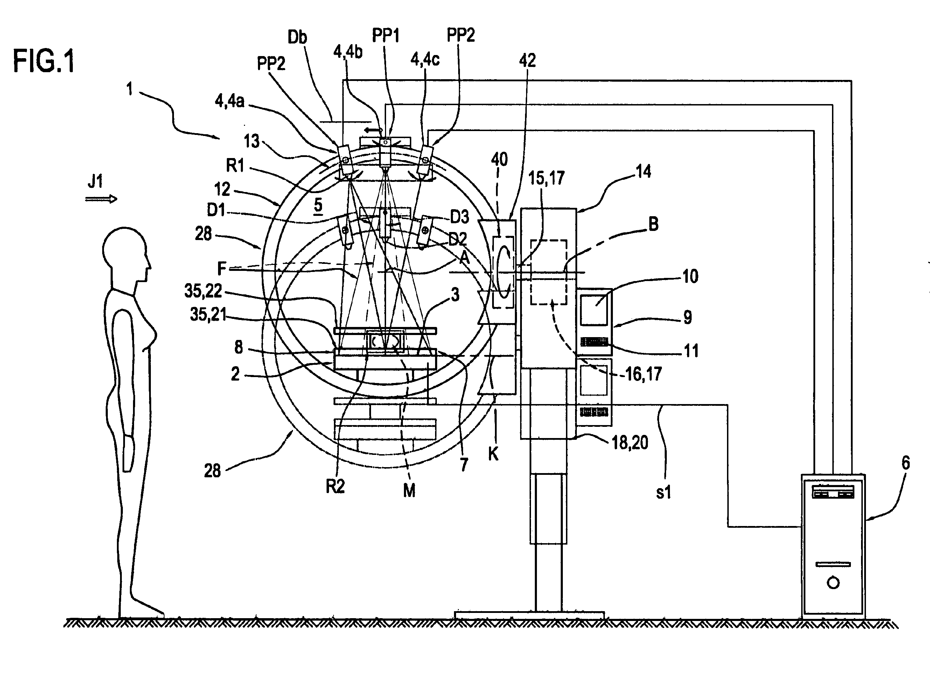

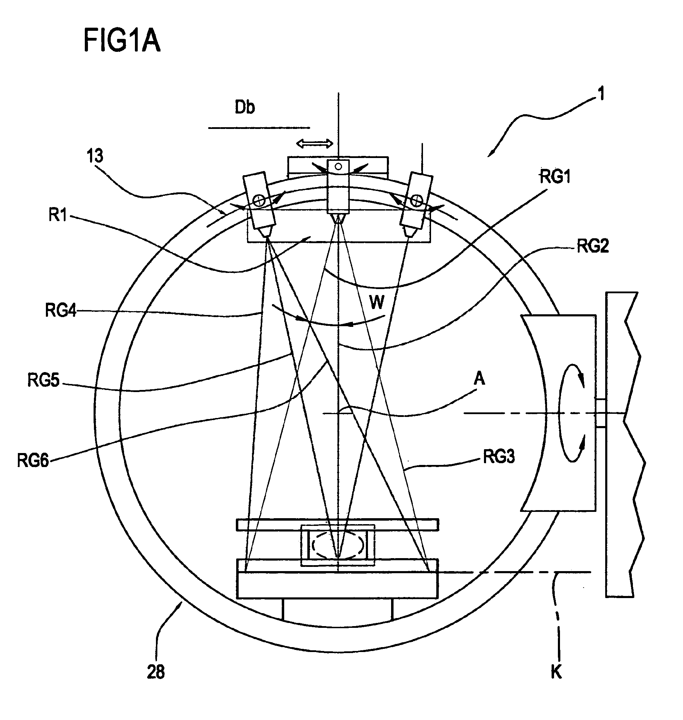

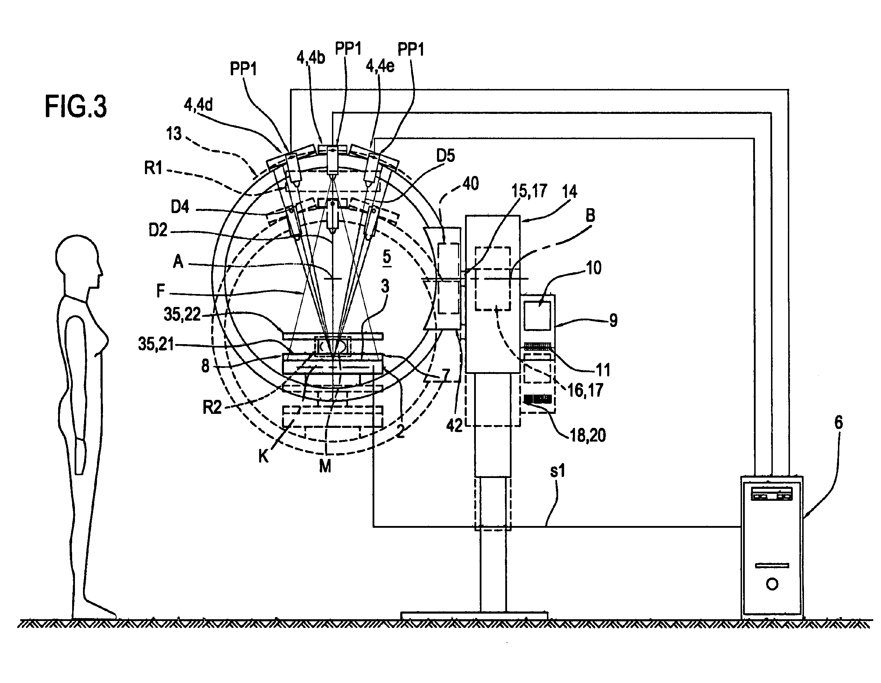

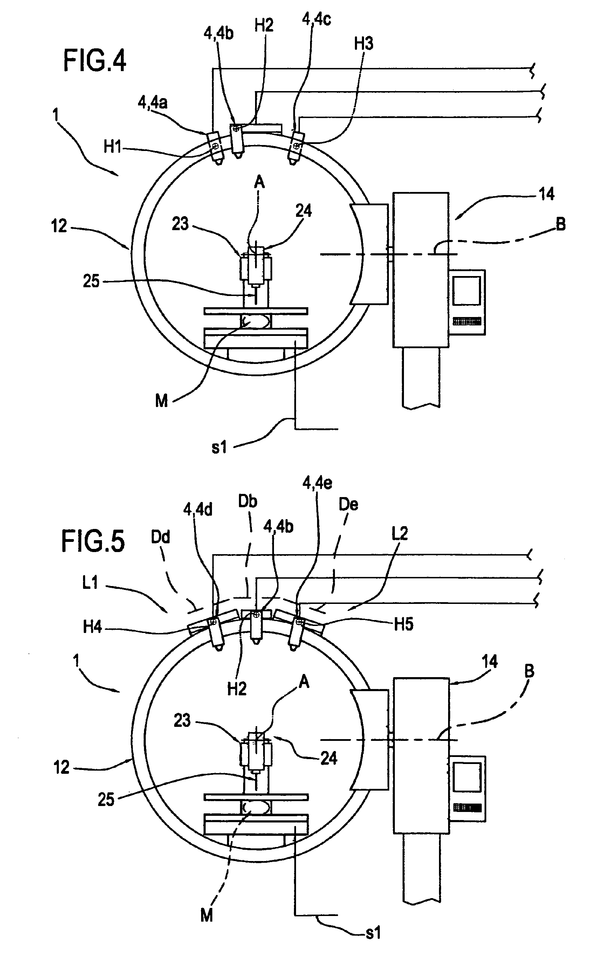

It should be noted that the term "X-ray tube" or just "tube" will hereinafter be used. This term must not however be understood as limiting the scope of the invention since the X-ray tube may, in variants of the apparatus 1 not illustrated, be substituted for X-ray sources of other kinds. Described below by way of example are two non-limiting embodiments of the apparatus 1 according to the invention. A first embodiment (Figures 1, 2 and 4) will be described first, followed by a second embodiment (Figures 3 and 5).

Preferably, but without limiting the invention, the first embodiment of the apparatus 1 comprises a tube 4b which is movable relative to the detector device 2 and tubes (4a, 4c) which are positioned stably (that is, which are fixed) at predetermined positions relative to the detector device 2.

It should be noted that in Figure 1, the tube which is movable relative to the detector device 2 is labelled 4b and the tubes which are fixed to the detector device 2 (hereinafter also referred to as "fixed tubes" or "stationary tubes") are labelled 4a and 4c.

In light of this, it should be noted that the apparatus 1 comprises a first part PP1 of tubes 4 which are movable relative to the detector device 2 (in the specific case, the first part comprises a movable tube 4b) and a second part PP2 of tubes which are positioned stably (that is, which are fixed) at predetermined positions relative to the detector device 2 (in the specific case, the second part comprises a tube 4a and a tube 4c).

It should also be noted that in the embodiment of the apparatus 1 illustrated in Figure 1, some (4a) of the fixed tubes 4 are positioned on one side L1 of the movable tube 4 and other tubes (4b) of the fixed tubes are positioned on the side L2 of the movable tube 4.

Each X-ray tube 4 is positioned and oriented in such a way as to emit a corresponding X-ray beam F towards the first, detection plane 3.

The X-ray beam F is preferably a beam of slightly diverging X-rays (that is, it is conical) directed principally along a corresponding principal direction of emission (D1, D2, D3).

It should be noted that the principal direction of emission or of maximum emission (D1, D2, D3) of each tube 4 defines a central axis of emission of the selfsame tube.

Indeed, as may be observed in Figure 1, each tube 4 emits a beam of X-rays according to an emission cone (whose opening angle γ1 and γ2 is adjustable in both directions).

In light of this, it should be noted that each X-ray tube 4 is preferably equipped with a beam collimating device which is configured to adjust the divergence of the beam relative to the principal direction of emission (D1, D2, D3).

It should be noted that in the first embodiment, the focal points of emission of the stationary X-ray tubes (4a, 4c) are positioned in the same second plane 5 at a right angle to the first, detection plane 3, and the tube 4b is movable in the selfsame second plane 5.

Preferably, the second plane 5 is parallel to the plane T defined by the patient's chest TO when the patient is correctly positioned relative to the apparatus 1 to perform tomosynthesis / mammography (that is, when the breast M is correctly positioned relative to the apparatus 1).

It should be noted that relative to the first, detection plane 3 the tubes (4a, 4b, 4c) are oriented according to a plurality of different angles, that is, the principal directions of emission (D1, D2, D3) differ from each other in orientation relative to the first, detection plane 3.

Each X-ray tube 4 is activatable individually to emit a corresponding X-ray beam F towards the first, detection plane 3.

It should also be noted that the movable tube 4b, hereinafter also referred to as first tube 4b, is movable, according to the invention, parallel to the first plane 3 of the detector device 2.

More specifically, it should be noted that the movable tube 4b is translated along a first direction of movement Db parallel to the first plane 3 of the detector device 2.

It should also be noted that the first direction of movement Db lies in the second plane 5 and, preferably, is parallel also to the plane T defined by the patient's chest TO.

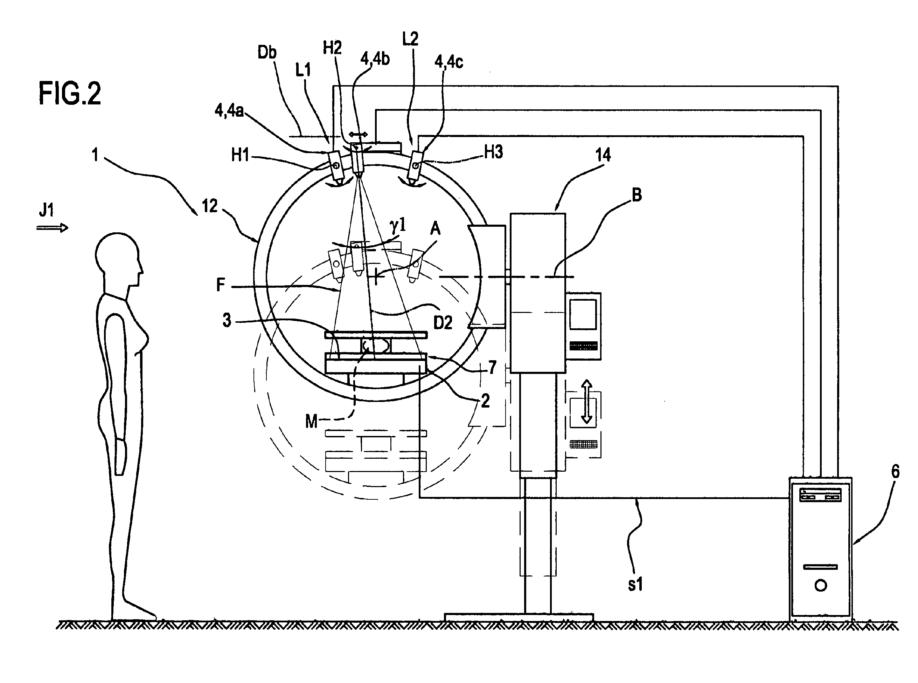

Figure 2 shows a limit position reached by the middle tube 4b (in this regard, it should be noted that in Figure 1 the middle tube 4b is at a central position, whereas in Figure 2 it is at a limit position on the left-hand side of the apparatus 1). It should be noted that advantageously, the middle tube 4b remains at the same distance from the first plane 3 at each of the positions it can reach during its movement along the direction of movement Db.

Advantageously, this makes it possible to keep the distance between the point of emission of the tube 4b and the plane 3 of the detector device 2 substantially unchanged at the plurality of emission positions of the tube 4b.

Described below is the second embodiment, illustrated in Figures 3 and 5.

It should be noted that in the second embodiment of it, the apparatus 1 comprises a plurality of movable tubes 4.

In other words, the first part PP1 of the tubes 4 comprises more than one movable tube 4.

In particular, it should be noted that the tubes 4 are movable in the same plane 5 at a right angle to the first plane 3 of the detector device. Preferably, the second plane 5 is parallel to the plane T defined by the patient's chest TO when the patient is correctly positioned relative to the apparatus 1 to perform tomosynthesis / mammography (that is, when the breast M is correctly positioned to receive the X-rays).

In Figure 3 three movable tubes 4 are shown: a first, middle tube 4b, a second tube 4d located on the right-hand side of the first, middle tube 4b and a second, left side tube 4e located on the left-hand side of the first, middle tube 4b.

It should be noted that the first, middle tube 4b is movable along a first direction of movement Db as described above with reference to the tube 4b of the first embodiment.

The second tube 4d and the third 4e are movable, respectively, along a second direction of movement Dd and a third direction De of movement which are set at an angle to the first direction of movement Db.

It should be noted that the second direction of movement Dd and the third De have opposite inclinations relative to the first direction of movement Db.

Preferably, the second direction of movement Dd and the third De have inclinations which are equal but opposite in sign relative to the first direction of movement Db.

Described below are some aspects common to both the first and the second embodiment.

It should be noted that preferably, but without limiting the invention, the movable tubes 4 are moved along a guide through the agency of respective drive means.

In light of this, it should be noted that the movable tubes 4 are driven in such a way as to allow the X-rays to be emitted from a plurality of positions along the respective direction of movement (which are preferably, but without limiting the invention, equally spaced).

Described below is a preferred embodiment of the mounting structure for supporting the tubes 4 and the detector device 2 and also forming part of the apparatus.

The apparatus 1 comprises a first frame 12 for supporting the X-ray tubes 4 and the detector device 2.

In the preferred embodiment, the first frame 12 has the shape of a ring. More specifically, it should be noted that in Figure 1, the centre of the ring 28 defined by the first frame 12 is labelled A.

In the first embodiment, the detector device 2 and the stationary X-ray tubes 4 are stably fixed to the first frame 12.

Thus, the stationary X-ray tubes 4 are positioned in a predetermined space relationship with the detector device 2.

Preferably, the stationary X-ray tubes 4 are supported by an arc 13 of the same ring 28.

In this regard, it should be noted that the stationary X-ray tubes 4 are preferably distributed along the arc 13 of the selfsame ring 28 defined by the first frame 12.

The detector device 2 is preferably, but without limiting the invention, positioned in the internal region of the ring 28 defined by the first frame 12.

As regards the position of the X-ray tubes (4a, 4b, 4c) in the first embodiment illustrated in Figures 1, 2 and 4, the following should be noted.

In the second embodiment, the directions of movement of the respective movable tubes (4b, 4d, 4e) are preferably tangent to the ring 28.

The apparatus 1 further comprises a second frame 14 and the first frame 12 is rotatably supported relative to the second frame 14.

More specifically, the first frame 12 is rotatable about an axis B (preferably horizontal) parallel to the second plane 5.

It should also be noted that, in the preferred embodiment, the axis B lies in the second plane 5 and, still more preferably, passes through the centre A of the ring 28.

In Figure 1, the reference numeral 15 denotes a mounting shaft for supporting the first frame 12 and the reference numeral 16 denotes means for rotationally driving the shaft 15, both forming part of the apparatus 1.

It should be noted that the mounting shaft 15 and the means 16 for rotationally driving the shaft 15 define means 17 for rotating the first frame 12 relative to the second frame 14.

It should also be noted that in the preferred embodiment, the first frame 12 is movable vertically relative to the second frame 14.

In effect, the second frame 14 comprises a telescopic portion 18 whose length may be varied to allow the first frame 12 to be lifted / lowered. The telescopic portion 18 and the related movement means (not illustrated) define means 20 for vertical movement of the first frame 12 relative to the second frame 14.

Figure 1 shows two different vertical positions of the first frame 12, one drawn with a dashed line and the other with a continuous line.

It should be noted that in variants not illustrated, the means 20 for vertical movement of the first frame 12 relative to the second frame 14 may comprise diverse devices or means for allowing the first frame 12 to move vertically relative to the second frame 14.

It should also be noted that according to another aspect, the ring 28 is supported rotatably about its own centre A (or, more generally speaking, about an axis perpendicular to the plane 5).

In effect, it should be noted that the ring 28 is rotatably coupled to an element 42 which is integral with the shaft 15. In other words, the ring 28 is rotatable about its own centre A relative to the second frame 14.

It should also be noted that the reference numeral 40 denotes means for rotating the ring 28 about the axis A and forming part of the apparatus.

The rotation means 40 are associated with the element 42.

According to this aspect, it is possible to rotate the ring 28 as one about its own centre A. This rotation causes both the tubes 4 and the detector device 2 to rotate about A.

According to this aspect, it is possible to obtain images with each of the tubes 4 with the ring 28 positioned at different angles of rotation about A corresponding to the breast M being compressed along different directions.

In short, it should be noted that the first frame 12 is supported rotatably by the second frame 14 about a first axis B and about a second axis A. The axes A and B are at right angles to each other. According to the invention, the apparatus 1 also comprises a processing and control unit 6.

The processing and control unit 6 is connected to the X-ray tubes 4 in order to activate them and is also connected to the detector device 2 in order to receive a signal s1 relating to the quantity of X-rays received by the detector device 2.

The processing and control unit 6 is designed to derive from the signal s1 received a radiographic image representative of the portion of the patient's body subjected to examination (that is, the breast M).

It should be noted that the radiographic image representative of the breast M subjected to mammography / tomosynthesis is obtained by suitably processing the signal s1.

It should also be noticed that the processing and control unit 6 controls the drive means of the movable tubes 4 in such a way as to produce a suitable translational movement of the tubes. Further technical and functional features of the processing and control unit 6 are described below. It should also be noted that the apparatus 1 comprises an operator interface 9 designed to display the results of mammography and/or tomosynthesis (for example, the radiographic image/images derived from the parameter/parameters correlated with the signal s1 detected) and to receive commands from the operator.

The interface 9 preferably comprises a display 10 and a keyboard 11 for entering commands or, alternatively, a display of the touch-screen type. According to another aspect, the apparatus 1 further comprises a first contact element 21 configured to support the patient's breast M on it, and a second breast M contact element 22 which is movable to allow compression, or squeezing, of the breast M between the first contact element 21 and the second contact element 22 itself.

The first contact element 21 and the second 22 together define means 35 for the stable positioning of the breast M interposed between the detector device 2 and the X-ray tubes 4 to allow the breast M to be stably positioned.

It should be noted that the expression "stable positioning of the breast" used herein means the breast M is held in a desired position relative to the detector device 2 while mammography / tomosynthesis is being performed.

It should also be noted that the two elements (21, 22) define a region or zone R2 for positioning the breast (that is, a region where the breast is positioned in such a way that the X-ray beams emitted by the tubes 4 pass through it).

It should be noted that in one variant, the means 35 for the stable positioning of the breast M may be movable relative to the detector device 2 (that is, relative to the frame 12), in such a way that the breast can be compressed in different positions.

It should also be noted that more generally speaking the positioning region R2 is a spatial region in which the breast can be positioned in such a way that the X-ray beams emitted by the tubes 4 can pass through it in order to perform mammography / tomosynthesis.

According to another aspect, the apparatus 1 comprises a movable biopsy device 23 for taking a sample of tissue from the breast M.

In a preferred embodiment, the biopsy device 23 comprises a pushing unit 24 movable in space and equipped with a needle 25 designed to be inserted into the breast M in order to take a biopsy of biological material, that is, organic tissue, from the breast M.

According to this aspect, before the biopsy of biological tissue is taken, at least two X-ray tubes 4 are activated individually (or one after the other) in order to detect the irradiation in the first plane 3 of the detector device 2. Preferably, but without limiting the scope of the invention, two X-ray tubes 4 are activated in any order. Still more preferably, the middle tube (4b), one of the side tubes, either right-hand or left-hand, (4a, 4c) are activated in any order.

The terms "right-hand" and "left-hand" refer to the accompanying drawings.

The processing and control unit 6 is designed to derive from the set of data detected by the detector device 2 and relating to irradiation in the first, detection plane 3, the exact position of the needle 25 relative to a defined region of the breast M under examination (this region being selectable by the operator or determinable by the unit 6 according to different criteria).

The processing and control unit 6 is also designed to determine a path to be followed by the needle 25 through the breast M in order to allow the needle 25 to take a sample of biological tissue from the region of the breast M under examination.

It should be noted that, more generally speaking, the apparatus 1 might also be configured to determine a path to be followed by the needle 25 through the breast M with two images only (one relating to the middle tube 4c and another relating to one of the side tubes).

The image relating to the middle tube 4c makes it possible to identify the positioning of the needle 25 in the plane 3 of the detector device 2, whilst the image relating to the side tube in combination with the image relating to the middle tube 4c makes it possible to identify by triangulation the position of the needle 25 relative to the region under examination.

From the above description, it may be inferred that the apparatus 1 according to the invention makes it possible to identify with extreme precision, reliability and speed the trajectory of the movable biopsy device 23 for taking a sample of tissue from the patient's breast M.

In effect, the possibility of sequentially activating the tubes 4 makes it possible to obtain extremely quickly the position of the needle 25 relative to the region of the breast M under examination and the path followed by the selfsame needle 25 through the breast M, no waiting time being necessary between the deactivation of one tube 4 (after detection of its irradiation in the first plane 3 of the detector device 2) and the activation of another tube 4, as is the case with prior art machines on account of the need to stabilize the position of the single X-ray source relative to the detector device.

This advantageously reduces the time needed to take the biopsy of breast tissue, thus reducing patient stress due to waiting for invasive action on the body and also guarantees an accurate and precise biopsy at the selected region.

Described hereinafter is the operation of the apparatus 1 of the invention according to the first embodiment of it, illustrated in Figures 1,2 and 4, with reference to performance of tomosynthesis and mammography.

This description shall not be construed as limiting the invention, since the apparatus 1 may be used according to different methods.

The breast M is placed in the positioning region R2.

It should be noted that the breast M, once placed in the positioning region R2, is compressed between the first contact element 21 and the second contact element 22 before mammography or tomosynthesis is performed.

Use of the apparatus 1 to perform tomosynthesis on the breast M entails activating individually and in any order each of the X-ray tubes 4 and detecting in the first, detection plane 3 the X-rays emitted by each tube 4.

It should be noted that the radiation emitted by each tube 4 passes through the patient's breast M. In the first embodiment, the middle tube 4b is moved while one of the other, stationary tubes (4a, 4c) is activated.

That advantageously reduces the total time needed by the apparatus 1 to collect all the data necessary for deriving the radiographic image. Indeed, the fact that the middle tube 4b is moved while one of the other tubes (4a, 4c) is being used advantageously means that the total time taken by the detector device 2 to acquire the necessary data is drastically reduced.

Also, advantageously, when one of the stationary tubes (4a, 4c) is deactivated (because the acquisition cycle relating to that tube is over) the middle tube 4b is already in the emission position relative to the detector device 2 (that is to say, it is no longer affected by any transient connected with the movement of the middle tube 4b itself, such as position fluctuations due to deceleration, position control by the drives, etc.).

This makes it possible to obtain a tomosynthesis image of high quality because being able to have tubes 4 which are in a stable position relative to the detector device 2 during the entire X-ray emission cycle (as described above, even the middle tube 4b is stably positioned during acquisition) means that during the detection of the radiation, there is no displacement (however small) of the X-ray beam F relative to the detection plane 3 which might deteriorate the quality of the projections necessary for the tomosynthesis reconstruction.

From the data set corresponding to the X-rays emitted by the tubes 4 and detected in the first, detection plane 3 (that is, from the signal s1), the processing and control unit 6 derives the reconstruction of the tomographic planes representing the internal, in-depth structure of the breast M.

These tomographic planes are used by the operator to diagnose the presence or absence of anomalies (for example, suspect tumour growths) in the deep tissue of the breast M.

The apparatus 1 also allows mammography to be performed. In this case, only one X-ray tube 4 is activated (preferably the middle tube, labelled 4b in Figure 1)

Use of the apparatus 1 to perform mammography entails activating one X-ray tube (for example, the middle tube 4b) and detecting in the first, detection plane 3 the X-rays which are emitted by the tube 4 and which pass through the patient's breast M.

From the data set corresponding to the X-rays received in the first, detection plane 3, the processing and control unit 6 derives a radiographic image.

It should be noted that in tomosynthesis, a set of distinct tomographic planes of the breast M can be obtained where any lesions can be identified more easily than with mammography where the same information is superposed in a single image.

In the same way as what is set out above, in the second embodiment of the apparatus 1, each movable tube 4 is moved during the activation of (that is, during the acquisition cycle related to) another tube 4. Thus, in exactly the same way as described above, the total time necessary for data acquisition by the detector device 2 is reduced. More specifically, this advantageously makes it possible to switch between one tube 4 and another without having to wait for one to be deactivated before another is activated.

In effect, advantageously, by the time one of the tubes 4 is deactivated, the next tube 4 to be activated in the preselected activation sequence is at the required position (that is to say, the effects of all the transients connected with movement are sure to be depleted).

Moreover, the second embodiment, too, allows the quality of the derived image to be improved. Furthermore, the second embodiment, having a plurality of movable X-ray sources 4, allows the overall dimensions of the apparatus to be reduced. It should be noted that according to an aspect common to both the first and second embodiment, each movable tube 4 can be moved in at least three different modes.

According to a first, preferred mode, the tube 4 is moved between a plurality of operating positions where the tube 4 itself is stopped and placed in a condition of substantial immobility during emission of the X-ray beam F.

According to a second mode, the tube 4 is moved with uniform rectilinear motion (according to this aspect, the tube 4 is not stationary during acquisition but its speed remains constant). According to a third mode, the tube 4 is moved in accordance with a law of motion which comprise a first, high speed and a second, reduced speed. According to this mode, the tube 4 is moved at the second, reduced speed at (or around) positions where the tube 4 is activated and emits the X-ray beam F and is moved at the first, high speed during movements between these positions (the high speed therefore allows it to move more rapidly, whilst the reduced speed reduces vibrations and improves the quality of the image derived).

According to yet another aspect, the detector device 2 may be movable along any trajectory. According to this aspect, the apparatus 1 comprises means for the movement of the detector device 2.

It should be noted that these movement means allow the detector device 2 to be moved relative to the first frame 12, that is, relative to the X-ray sources.

Preferably, the detector device 2 is movable along the direction labelled K in Figure 1. Alternatively or in combination, the detector device 2 may be configured to rotate about an axis parallel to the axis A (preferably lying in the detection plane 3).

It should be noted according to this aspect, that the unit 6 is configured preferably to activate the movement of the detector device 2 during acquisition of the radiographic images for the biopsy or tomosynthesis examination.

According to another aspect, common to both the first and second embodiment, each movable tube 4 is rotated about a respective axis (H2, H4, H5) at a right angle to the second plane 5 during its movement along the respective direction of movement (Db, Dd, De).

Preferably, the axis of rotation (H2, H4, H5) of each tube 4 is proximal to the tube itself and, still more preferably, passes through the point of emission of the tube 4.

According to this aspect, the rotation of the tubes 4 makes it possible to maximize the uniformity of the energy fluency of each tube 4 in the plane 3 of the detector device 2.

It should be noted that, preferably, the axis of rotation of each tube is substantially at a right angle to the plane T defined by the patient's chest TO when the patient is correctly positioned relative to the apparatus 1 to perform mammography / tomosynthesis.

It should also be noted that the rotation of each tube 4 allows variation of the inclination of the principal direction of emission of the tube itself relative to the first plane 3 of the detector device 2.

It should be noted, furthermore, that in the first embodiment, also the stationary tubes (4a, 4c) may each be rotated about a respective axis (H1, H3) at a right angle to the second plane 5 (and preferably proximal to the tube itself). This rotation allows the respective irradiation in the first plane 3 to be made uniform among the plurality of tubes 4.

As is known, the energy fluency or radiation intensity of an X-ray source per unit area, measured on the detector device 2 relative to the central axis, varies inversely to the square of the distance between the X-ray source and the detector device 2.

It is also known that the relative energy fluency of a tube 4 in a surface which is arbitrarily small and located at a given distance from the tube 4 varies with the angle formed between the ray incident upon the surface itself and the central axis of emission of the selfsame tube 4 (by way of an example, this angle is labelled W in Figure 1A). More specifically, the energy fluency is at its maximum along the direction of the central axis (D1, D2, D3) and decreases with distance away from the central axis

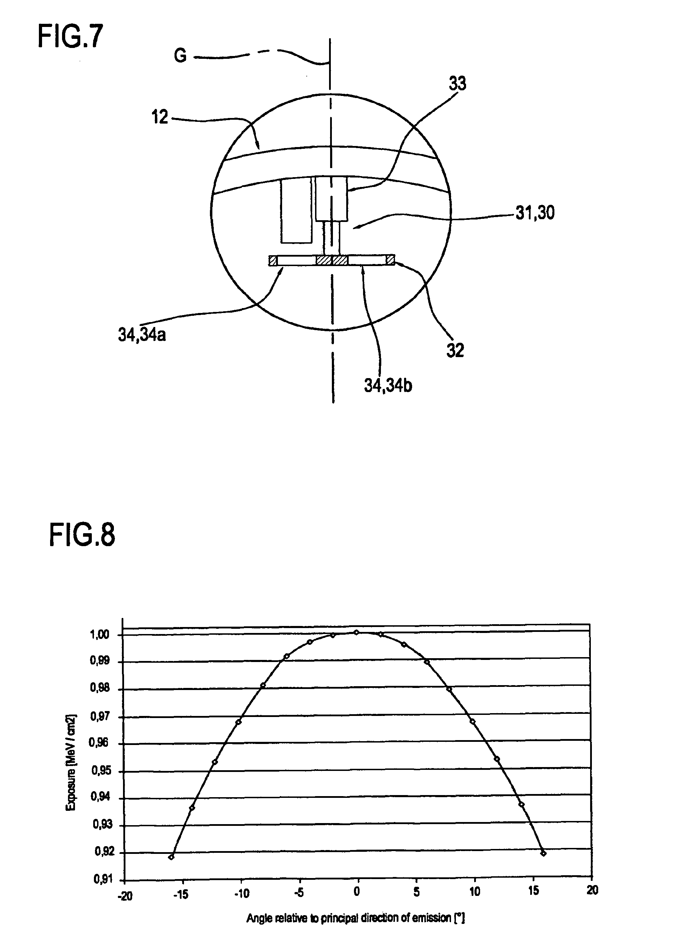

By way of an example, Figure 8 shows the energy fluency curve at a given distance from the point of emission of a tube 4 as a function of the angle formed between a ray and the central axis.

In Figure 1A, the reference labels RG1, RG2, RG3 denote three X-rays from the tube 4b and the reference labels RG4, RG5, RG6 denote three X-rays from the tube 4a

With reference to the tube 4a and by way of an example, it should be observed that on account of rotation of the tube 4a about H1, the ray RG4 travels, from the focal point of emission, a smaller distance compared to the ray RG5 from the plane 3 to strike the detector device 2 but, on the other hand, is positioned at a larger angle to the central axis of the tube 4a (which in practice coincides with the ray RG5). Thanks to these opposite effects, it is possible to choose a direction of the central axis D1 such that in the detection plane 3, the uniformity of energy fluency is maximized between the rays RG5 and RG4 (indeed, the fact that the distance of the ray RG4 is smaller than RG5 would create a greater fluency in the plane 3 but this effect is reduced or cancelled out by the fact that RG4 is positioned at an angle greater than RG5 relative to the central axis).

In exactly the same way, the above also applies to all the other rays / tubes 4.

Thus, advantageously, the rotation of each tube 4 about the respective axis (H1, H2, H3) makes it possible to even out, that is, to make uniform, the energy fluency in the plane 3 of the detector device 2 (of each tube 4 and/or of the different tubes relative to one another)

Described below are other aspects common to both the first and the second embodiment of the apparatus 1.

According to yet another aspect, the apparatus 1 comprises means 30 for adjusting the spectrum of the X-ray beam F, associable with one or more of the X-ray tubes 4, to allow adjustment of the X-ray beam F emission spectrum of the tube 4 the selfsame means 30 are associated with.

It should be noted that the term "emission spectrum" is used to mean the distribution of the X-ray beam intensity as a function of the frequencies.

In a preferred embodiment of the means 30 for adjusting the spectrum of the X-ray beam F, illustrated in Figure 7, the means 30 comprise a device 31, associable with a corresponding X-ray tube 4, comprising a beam attenuation filter 34 (preferably, three filters, two of which 34a and 34b are illustrated in Figure 7), a filter support 32, and means 33 for moving the filters 34 between a non-operating position D and an operating position E of the selfsame filters.

It should be noted that each filter 34 is in the operating position E when the X-ray beam F emitted by the tube 4 the device 31 itself is associated with passes through it and, vice versa, is in the non-operating position D when the X-ray beam F does not pass through it. That way, in the operating position E and depending on its properties (geometrical and relating to the material it is made of) the filter 34 allows portions of the beam F spectrum to pass through selectively (that is to say, the beam F spectrum upstream and downstream of the filter 34 can be modified and the spectrum itself is thus adjusted).

In the embodiment of the device 31 of Figure 7, the support 32 comprises a carousel, adapted to rotate about an axis G, and two filters 34a and 34b mounted on the periphery of the carousel.

The means 33 for moving the filters 34 are configured to allow the carousel to rotate.

In this embodiment of the filtering device, the rotational drive of the carousel causes a filter 34 to move from the non-operating position D to the operating position E and vice versa.

It should be noted that, preferably, each tube 4 has a device 31 associated with it, as described above.

In another embodiment not illustrated, the means 30 for adjusting the spectrum of the beam F of one or more of the X-ray tubes 4 comprise an electronic adjustment stage configured to allow the polarization voltage of one or more of the X-ray tubes 4 to be adjusted.

In this embodiment, adjustment of the polarization voltage of one or more of the X-ray tubes 4 allows adjustment of the emission spectrum of the X-ray tube 4 and/or tubes 4 which the electronic adjustment stage is associated with.

It should be noted that in any of the above described embodiments of the means 30 for adjusting the spectrum of the beam F of one or more of the X-ray tubes 4, the processing and control unit 6 is configured to control the means 30 for adjusting the emission spectrum in such a way as to adjust the emission spectrum of the tube / tubes 4 in a first and in a second configuration corresponding to the emission of a beam F with a first and a second, different spectrum, respectively.

The processing and control unit 6 is configured to derive, from the data set detected by the detector device 2 with the tube/tubes 4 in the first and in the second configuration, information regarding the nature of the internal tissue of the breast M.

In effect, it should be noted that for each X-ray tube 4, the attenuation variation of beam intensity by a tissue part with the variation of the X-ray spectrum depends on the nature of the tissue which the X-rays pass through.

It follows that the relative contrast, meaning here the contrast variation in the same region between the two images (that is, between a first radiographic image derived when the tube 4 emits a beam according to the first spectrum and a second radiographic image derived when the tube 4 emits a beam according to the second spectrum), depends on the nature of the tissue of the breast M.

By way of an example, the relative contrast between the radiographic images derived in the above mentioned first and second configurations is different for a region of the breast M where there is glandular tissue compared to a region where there is a tumour growth.

Thus, the unit 6 is programmed with a mathematical algorithm such as to enhance the nature of the breast tissue under examination, according to the two images relating to the first and second configurations.

This aspect therefore allows the capabilities of the apparatus 1 both in tomosynthesis and in mammography to be enhanced, while maintaining an extremely high speed.

It should also be noted that this aspect allows the capabilities of the apparatus 1 both in tomosynthesis and in mammography to be enhanced through the combined use of the spectrum adjustment means and methods for subtracting the images obtained with different spectra (in this regard, it should be noted that the processing and control unit is programmed to implement these subtraction methods).

It should be noted that what is stated above means implementing techniques known as "dual energy techniques" (such as dual energy mammography or contrast-enhanced digital mammography).

The non-linear combination of different energy images (that is images with different spectra) allows production of a hybrid image - whether of mammography or of tomographic planes, depending on the data acquisition technique used - in which the contrasts of relevant structures are maintained while those of normal structures are significantly attenuated.

"Dual energy techniques" also allow the contrast between two materials to be cancelled in order to intensify the contrast between a third material and the other two.

That means that assuming, for example, that a mammary organ is made up of three types of tissue, namely glandular, adipose and cancerous tissue, the cancellation or minimization of the contrast between healthy tissue types may facilitate detection of damaged tissue.

In connection with subtraction methods, it should be noted that the two images subtracted are acquired preferably without making the patient change position (that is, keeping the breast compressed).

Also in connection with the subtraction of planar images, it should be noted that the images are acquired from the same relative position between source and detector device 2.

It should be noted that in tomosynthesis it is in any case possible to acquire two sets of projections in different positions of the source (relative to the detector device). According to this aspect, subtraction is performed in reconstructed tomographic planes (provided the orientation of the planes relative to the patient's breast is the same).

It should be noted that the above described embodiments of the means 30 for adjusting the spectrum of the X-ray beam F may be alternative to or combinable with each other. Indeed, a combination of them in the same apparatus 1 is possible.

According to a further aspect, the apparatus 1 comprises a grid 7 for removing diffuse radiation. The grid 7 for removing diffuse radiation is interposed between the X-ray tubes 4 and the detector device 2, that is, it is located along the path of the X-ray beam F so as to intercept the beam F.

More specifically, while mammography or tomosynthesis is being performed or while a biopsy is being taken (that is, during use of the apparatus 1) the grid 7 for removing diffuse radiation is interposed between the patient's breast M and the detector device 2.

It should be noted that the grid 7 for removing diffuse radiation defines a space 8 for removing diffuse radiation configured to allow only X-rays having predetermined angles (that is, primary radiation) to pass through it.

It should be noted that hereinafter, the radiation corresponding to the X-ray beam F emitted by one of the X-ray tubes 4 will also be referred to as "primary radiation" while the photons deviated by the X-rays passing through a portion of the human body (that is, the breast M) and propagating in different directions relative to the primary radiation will be referred to as "scattering radiation" or "diffuse radiation" or "secondary radiation".

Below is a detailed description of the grid 7 for removing diffuse radiation, forming part of the apparatus 1 according to the invention.

The grid 7 for removing diffuse radiation comprises a plurality of plates 26 which are positioned to face one another, that is, which are positioned in such a way that their respective large planar faces or planes 27 are opposed to each other.

It should be noted that the term "plate" is used to mean a substantially planar element characterized by a longitudinal direction, that is a direction of principal extension, a transverse direction and a thickness.

Preferably, the plates 26 are in the form of laminas (whose thickness is in the order of hundredths of a millimetre).

It should be noted that the plates 26 together define the space 8 for removing the diffuse radiation.

The plates 26 converge towards the same region R1 proximal to one of the X-ray tubes 4 (preferably towards a small-volume region).

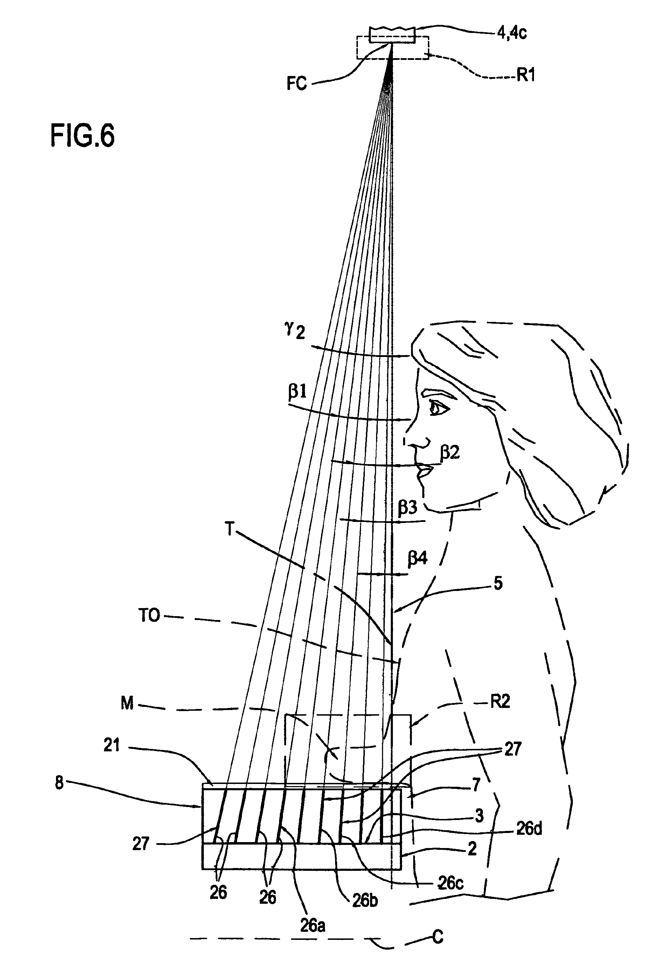

It should be noted that in the side view of the apparatus 1 shown in Figure 6, the plates 26 converge towards the same point of convergence FC, that is, towards the same focus.

It should also be noted that one of the tubes 4 of the apparatus 1 is positioned at the point of convergence FC, or focus (in the embodiment illustrated, the middle tube 4c).

In other words, the plates 26 are oriented in such a way that the planes 27 defined by the large faces of the selfsame plates 26 are, in use, slightly rotated about the plane T defined by the patient's chest TO to converge at the same point of convergence or focus FC.

Preferably, the inclination of the plates 26 is a function of their distance from the second plane 5. More specifically, as may be observed in Figure 6, the inclination of the planes 27 defined by the large faces relative to the plane T defined by the patient's chest increases with distance of the plates 26 from the second plane 5 (that is, the greater the distance of the plates 26 from the second plane 5, the greater the inclination, and the smaller the distance of the plates 26 from the second plane 5, the smaller the inclination). Figure 6 schematically illustrates a portion of the grid 7 showing a plurality of plates 26. For convenience, four of the plates are labelled individually 26a, 26b, 26c, 26d, starting from the one of the four that is furthest from the second plane 5.

It should be noted that each of the plates (26a, 26b, 26c, 26d) has its own angle of inclination (β1, β2, β3, β4) relative to the plane T defined by the patient's chest TO and that these angles of inclination (β1, β2, β3, β4) decrease, starting from the plate 26a, towards the second plane 5. More specifically, in Figure 6, the planes 27 defined by the large faces of the plates (26a, 26b, 26c, 26d) converge, according to the invention, at a focus point labelled FC (in three-dimensional space, that point is a straight line).

It should be noted that the grid 7 for removing diffuse radiation is a "focused" grid (that is, the plates are arranged in such a way as to converge towards the same point of convergence or focus FC). It is stressed that one of the tubes 4 of the apparatus 1 is positioned at the point of convergence FC, or focus (preferably, the middle tube 4c).

Thus, more generally speaking and according to the invention, the plates 26 are oriented in such a way as to converge at the point of emission of one of the X-ray tubes 4.

According to the invention, the plates 26 extend along a direction of extension K substantially parallel to a plane T defined by the patient's chest TO when the patient's breast M is in the positioning region R2 (that is, when the patient is in the correct position for mammography /tomosynthesis to be performed).

It should be noted that patients are correctly positioned when he/she faces the second plane 5 in front of him/her (in Figure 1, on the other hand, the patient is turned by 90° to the correct position, that is, he/she is positioned sideways relative to the second plane 5).

It should also be noted that Figure 6 schematically illustrates a breast M which is positioned correctly in the positioning region R2.

It should be noted, furthermore, that the direction of extension K is also substantially parallel to the second plane 5, that is, the plane in which the X-ray source/sources is/are movable, that is in which the X-ray source/sources is/are positioned. Moreover, in the preferred embodiment, the second plane 5 is substantially at a right angle to the first plane 3.

It should be borne in mind that the plates 26 are positioned in such a way as to substantially face towards the patient's chest TO.

It should be noted that the apparatus 1 comprises movement means (not illustrated) for the grid 7 for removing diffuse radiation, by which the grid 7 is moved at least during emission of the X-ray beam. Preferably, these movement means are configured to allow the grid 7 for removing diffuse radiation to be translated along a direction of translation C at a right angle to the direction of extension K.

It should be noted that the direction of translation C is preferably parallel to the first, detection plane 3.

Also, preferably and in the preferred embodiment illustrated in Figure 1, the direction of translation C is a direction substantially at a right angle to the second plane 5.

Preferably, the movement means are activated to move the grid 7 for removing diffuse radiation while the mammography or even tomosynthesis is being performed or a biopsy is being taken, in such a way as to cancel the shadow produced by the plates on the detector device 2 (and hence to reduce the artificial effect it produces on the radiographic image).

According to another aspect, the grid 7 for removing diffuse radiation is characterized by a relatively high plate density along the direction C. Preferably, along the direction labelled C, the grid 7 for removing diffuse radiation comprises a linear plate density per linear centimetre that is substantially greater than half the linear pixel density of the detector device 2 along the same direction.

According to this aspect and more generally speaking, the grid 7 comprises, along the direction C, a number of plates 26 per centimetre which is substantially greater than half the number of pixels per centimetre of the detector device 2 along the same direction C.

The high plate density advantageously makes it possible to minimize the stroke of the grid 7 for removing diffuse radiation along the direction C needed to allow the shadow of the plates 26 in the first, detection plane 3 to be cancelled.

More specifically, it should be noted that a linear plate density along the direction C that is substantially greater than half the linear pixel density per linear centimetre of the detector device 2 along the direction C makes it possible to cancel the shadow caused by the presence of the grid 7 for removing diffuse radiation with a limited grid 7 stroke.

This advantageously makes it possible to minimize the field of vision loss along the direction labelled C in Figure 6.

For example, if the device has a pixel whose linear dimension is in the order of 0.1 mm and a plate density greater than 130 pairs per centimetre is chosen, the stroke of the grid 7 needed to correctly cancel the shadow on the image is less than two millimetres.

The plates 26 are made preferably of a metal with a high atomic number, such as lead, for example, or in any case of a material capable of attenuating X-rays.

The plates 26 are configured in such a way as to allow a large fraction of the primary radiation to pass through the space 8 for removing diffuse radiation and to reduce the amount of secondary radiation passing through the space 8 for removing diffuse radiation.

The filtering grid 7 therefore advantageously attenuates the secondary radiation incident upon the first, detection plane 3, and at the same time allows the primary radiation to pass through the space for removing diffuse radiation.

It should be noted that, advantageously, the grid 7 for removing diffuse radiation of the apparatus 1 does not need to be removed during tomosynthesis and in fact considerably improves the quality of the radiographic image even during tomosynthesis. More specifically, the grid 7 for removing diffuse radiation makes it possible, during both mammography and tomosynthesis, to increase the ratio between the contrast of the radiographic image detected with the grid 7 for removing diffuse radiation and without the grid 7.

An advantage of this aspect is that it allows both tomosynthesis and mammography to be performed without having to remove the grid 7.

It should be noted that the apparatus 1 in any case contemplates the possibility of removing the grid 7 manually if focusing does not allow a good quality image to be obtained.

Alternatively, in a variant which is not illustrated, the apparatus 1 comprises means for moving the grid 7 between an operating position and a non-operating position to allow the grid to be removed if focusing does not allow a good quality image to be obtained.

The invention described is susceptible of industrial application and may be modified and adapted in several ways without thereby departing from the scope of the inventive concept. Moreover, all details of the invention may be substituted for technically equivalent elements.

## Claims

1. An apparatus (1) for performing tomosynthesis and mammography of a breast (M) of a patient, said apparatus comprises:
- an X-ray detector device (2), designed to receive and detect X-rays in a first, detection plane (3);
- a plurality of X-ray sources (4), positioned in a second plane (5) which is substantially at a right angle to the first plane (3) and which X-ray sources (4) can be individually activated for emitting a corresponding X-ray beam (F) towards the first, detection plane (3),
- a region (R2) for positioning the breast (M);
- a processing and control unit (6), connected to the X-ray sources (4) for activating them individually and connected to the detector device (2) for receiving a signal (s1) relating to the X-rays which passed through the breast (M) and were detected by the detector device (2), the unit (6) being designed to derive from the signal (s1) at least one radiographic image representative of the internal structure of the breast (M) of the patient, **characterized in that** at least a first part (PP1) of the sources (4) is able to move relative to the detector device (2) and comprising a first source (4b) mobile in a first direction (Db) of movement parallel with the first, detection plane (3) for emitting X-rays from a plurality of operating positions along said first direction (Db) of movement.

2. The apparatus according to claim 1, wherein the sources (4) of a second part (PP2) of the plurality of sources (4) are stably fixed relative to the detector device (2) and are positioned on one side (L1) and on the other side (L2) of the first source (4b) for emitting the X-rays towards the first, detection plane (3) according to a plurality of different angles.

3. The apparatus according to claim 1 or 2, wherein the first part (PP1) of the sources (4) also comprises a second source (4d) and a third source (4e) which are positioned in such a way that they can move respectively on one side (L1) and on the other side (L2) of the first tube (4b) and are able to move respectively in a second direction (Dd) and a third direction (De) of movement, said directions being set at an angle to the first direction (Db).

4. The apparatus according to any of the foregoing claims, wherein the mobile sources (4) are designed to rotate about an axis (H4, H2, H5) which is at a right angle to the second plane (5) depending on their position along the respective direction of movement (Dd, Db, De).

5. The apparatus according to any of the foregoing claims, wherein the direction or directions of movement (Dd, Db, De) of the mobile source or sources (4) are substantially parallel with a plane (T) defined by the chest (TO) of the patient when the breast (M) is positioned in the positioning region (R2).

6. The apparatus according to any of the foregoing claims, wherein the sources (4) are positioned along an arc (13).

7. The apparatus according to any of the claims from 1 to 6, wherein the apparatus (1) comprises a first frame (12) having the shape of a ring (28) which supports the X-ray sources (4) and the detector device (2).

8. The apparatus according to claim 7, **characterized in that** it comprises a second frame (14) and also being **characterized in that** the first frame (12) is rotatably supported by the second frame (14).

9. The apparatus according to claim 8, **characterized in that** it comprises means (20) for vertical movement of the first frame (12) relative to the second frame (14), for allowing a vertical movement of the first frame (12).

10. The apparatus according to any of the claims from 5 to 7, **characterized in that** the first frame (12) can rotate about a centre (A) of the ring (28).

11. The apparatus according to any of the foregoing claims, **characterized in that** it comprises a grid (7) for removing diffuse radiation, the grid (7) being interposed between the positioning region (R2) and the detector device (2) so that the X-rays which passed through the breast (M) pass through it, the grid (7) for removing diffuse radiation comprising a plurality of plates (26) which are positioned opposite the patient's chest (TO) when the breast (M) is positioned in the positioning region (R2), said plates being angled in such a way that they converge towards the same region (R1) proximal to one of the X-ray sources (4).

12. The apparatus according to claim 11, **characterized in that** the grid (7) for removing diffuse radiation comprises, in a direction (C) at a right angle to the chest (TO) of the patient, a number of plates (26) per centimetre which is greater than half of a number of pixels per centimetre of the detector device (2) in that direction (C).

13. The apparatus according to claim 11 or 12, **characterized in that** it comprises means for moving the grid (7) which are designed to allow a movement of the grid (7) in a direction (C) of movement substantially at a right angle to a direction (K) of extension of the plates (26).

14. The apparatus according to any of the foregoing claims, **characterized in that** it comprises means (30) for adjusting the spectrum of the X-ray beam (F), associable with at least one of the X-ray sources (4), and also being **characterized in that** the processing and control unit (6) is designed to control the adjusting means (30), allowing adjustment of the spectrum of the beam (F) emitted by the source (4) between a first emission spectrum and a second emission spectrum, and for deriving an image from the X-rays detected in the first, detection plane (3) and relating to the first and second emission spectra.

15. The apparatus according to claim 14, wherein the adjusting means (30) comprise at least one attenuating filter (34) for the beam and means (33) for moving the filter (34) between an operating position (E) in which the filter (34) acts on the beam (F) to define the first emission spectrum and a non-operating position (D) in which the filter (34) does not act on the beam (F), thus defining the second emission spectrum.

## Patentansprüche

1. Vorrichtung (1) zur Durchführung einer Tomosynthese und Mammographie der Brust (M) eines Patienten, wobei diese Vorrichtung umfasst:
- ein Röntgenstrahlenerfassungsgerät (2), ausgelegt, um Röntgenstrahlen in einer ersten Erfassungsebene (3) zu empfangen und zu erfassen;
- eine Vielzahl von Röntgenstrahlenquellen (4), positioniert in einer zweiten Ebene (5), die im Wesentlichen rechtwinklig zur ersten Ebene (3) angeordnet ist, wobei diese Röntgenstrahlenquellen (4) individuell aktiviert werden können, um einen entsprechenden Röntgenstrahl (F) in Richtung der ersten Erfassungsebene (3) abzugeben;
- eine Region (R2) zum Positionieren der Brust (M);
- eine Verarbeitungs- und Steuerungseinheit (6), verbunden mit den Röntgenstrahlenquellen (4) zu deren individuellen Aktivierung und verbunden mit dem Erfassungsgerät (2) zum Empfangen eines Signals (s1), das sich auf die Röntgenstrahlen bezieht, die durch die Brust (M) drangen und vom Erfassungsgerät (2) erfasst wurden, wobei die Einheit (6) so ausgelegt ist, dass sie vom Signal (s1) mindestens ein Röntgenbild ableitet, das repräsentativ für die interne Struktur der Brust (M) des Patienten ist, **dadurch gekennzeichnet, dass** mindestens ein erster Teil (PP1) der Quellen (4) in der Lage ist, sich relativ zum Erfassungsgerät (2) zu bewegen, und umfassend eine erste Quelle (4b), die in eine erste Bewegungsrichtung (Db) beweglich ist, die parallel zur ersten Erfassungsebene (3) verläuft, um Röntgenstrahlen aus einer Vielzahl von Betriebspositionen entlang der ersten Bewegungsrichtung (Db) abzugeben.

2. Vorrichtung nach Anspruch 1, wobei die Quellen (4) eines zweiten Teils (PP2) der Vielzahl von Quellen (4) fest relativ zum Erfassungsgerät (2) befestigt und auf einer Seite (L1) und auf der anderen Seite (L2) der ersten Quelle (4b) positioniert sind, um Röntgenstrahlen zur ersten Erfassungsebene (3) gemäß einer Vielzahl unterschiedlicher Winkel abzugeben.

3. Vorrichtung nach Anspruch 1 oder 2, wobei der erste Teil (PP1) der Quellen (4) ebenfalls eine zweite Quelle (4d) und eine dritte Quelle (4e) umfasst, die so positioniert sind, dass sie sich jeweils auf eine Seite (L1) und auf die andere Seite (L2) der ersten Röhre (4b) bewegen können und in der Lage sind, sich jeweils in eine zweite Bewegungsrichtung (Dd) und eine dritte Bewegungsrichtung (De) zu bewegen, wobei diese Richtungen in einem Winkel zur ersten Richtung (Db) festgelegt sind.

4. Vorrichtung nach einem der vorhergehenden Ansprüche, wobei die beweglichen Quellen (4) ausgelegt sind, um sich um eine Achse (H4, H2, H5) zu drehen, die an einem rechten Winkel der zweiten Ebene (5) angeordnet ist, abhängig von ihrer Position entlang der jeweiligen Bewegungsrichtung (Dd, Db, De).

5. Vorrichtung nach einem der vorhergehenden Ansprüche, wobei die Bewegungsrichtung oder die Bewegungsrichtungen (Dd, Db, De) der beweglichen Quelle oder Quellen (4) im Wesentlichen parallel zu einer Ebene (T) verläuft/verlaufen, die vom Brustkorb (TO) des Patienten definiert ist, wenn die Brust (M) in der Positionierungsregion (R2) positioniert ist.

6. Vorrichtung nach einem der vorhergehenden Ansprüche, wobei die Quellen (4) entlang eines Bogens (13) positioniert sind.

7. Vorrichtung nach einem der Ansprüche 1 bis 6, wobei die Vorrichtung (1) einen ersten Rahmen (12) umfasst, aufweisend die Form eines Rings (28), der die Röntgenstrahlenquellen (4) und das Erfassungsgerät (2) trägt.

8. Vorrichtung nach Anspruch 7, **dadurch gekennzeichnet, dass** sie einen zweiten Rahmen (14) umfasst und ferner **dadurch gekennzeichnet, dass** der erste Rahmen (12) drehbar vom zweiten Rahmen (14) getragen wird.

9. Vorrichtung nach Anspruch 8, **dadurch gekennzeichnet, dass** sie Mittel (20) für die vertikale Bewegung des ersten Rahmens (12) relativ zum zweiten Rahmen (14) umfasst, um eine vertikale Bewegung des ersten Rahmens (12) zu erlauben.

10. Vorrichtung nach einem der Ansprüche 5 bis 7, **dadurch gekennzeichnet, dass** der erste Rahmen (12) sich um ein Zentrum (A) des Rings (28) drehen kann.

11. Vorrichtung nach einem der vorhergehenden Ansprüche, **dadurch gekennzeichnet, dass** sie ein Raster (7) zur Entfernung diffuser Strahlung umfasst, wobei das Raster (7) zwischen der Positionierungsregion (R2) und dem Erfassungsgerät (2) angeordnet ist, sodass die durch die Brust (M) gedrungenen Röntgenstrahlen durch es dringen, wobei das Raster (7) zur Entfernung diffuser Strahlung eine Vielzahl von Platten (26) umfasst, die dem Brustkorb (TO) des Patienten gegenüberliegend angeordnet sind, wenn die Brust (M) in der Positionierungsregion (R2) positioniert ist, wobei diese Platten winkelförmig so angeordnet sind, dass sie zur selben Region (R1) proximal zur Röntgenstrahlenquelle (4) zusammenlaufen.

12. Vorrichtung nach Anspruch 11, **dadurch gekennzeichnet, dass** das Raster (7) zur Entfernung diffuser Strahlung in einer Richtung (C) rechtwinklig zum Brustkorb (TO) des Patienten eine Menge an Platten (26) pro Zentimeter umfasst, die größer ist als die Hälfte der Pixelzahl pro Zentimeter des Erfassungsgeräts (2) in dieser Richtung (C).

13. Vorrichtung nach Anspruch 11 oder 12, **dadurch gekennzeichnet, dass** sie Mittel zum Bewegen des Rasters (7) umfasst, die ausgelegt sind, um eine Bewegung des Rasters (7) in eine Bewegungsrichtung (C) zu ermöglichen, die im Wesentlichen rechtwinklig zu einer Ausdehnungsrichtung (K) der Platten (26) verläuft.

14. Vorrichtung nach einem der vorhergehenden Ansprüche, **dadurch gekennzeichnet, dass** sie Mittel (30) zum Einstellen des Spektrums des Röntgenstrahls (F) umfasst, kombinierbar mit mindestens einer der Röntgenstrahlenquellen (4), und ferner **dadurch gekennzeichnet, dass** die Verarbeitungs- und Steuerungseinheit (6) ausgelegt ist, um die Einstellmittel (30) zu steuern, um die Einstellung des Spektrums des von der Quelle (4) abgegebenen Strahls (F) zwischen einem ersten Emissionsspektrum und einem zweiten Emissionsspektrum zu ermöglichen und ein Bild der in der ersten Erfassungsebene (3) erfassten Röntgenstrahlen abzuleiten und mit dem ersten und dem zweiten Emissionsspektrum zu verknüpfen.

15. Vorrichtung nach Anspruch 14, wobei die Einstellmittel (30) mindestens einen Abschwächungsfilter (34) für den Strahl umfassen sowie Mittel (33) zum Bewegen des Filters (34) zwischen einer Betriebsposition (E), in der der Filter (34) auf den Strahl (F) wirkt, um das erste Emissionsspektrum zu definieren, und einer Nichtbetriebsposition (D), in der der Filter (34) nicht auf den Strahl (F) wirkt, wodurch das zweite Emissionsspektrum definiert wird.

## Revendications

1. Appareil (1) permettant d'effectuer la tomosynthèse et la mammographie d'un sein (M) d'une patiente, ledit appareil comprenant :
- un dispositif détecteur à rayons X (2), conçu pour recevoir et détecter des rayons X dans un premier plan de détection (3) ;
- une pluralité de sources de rayons X (4), positionnées dans un second plan (5) étant substantiellement orthogonal par rapport au premier plan (3) et dont les sources de rayons X (4) peuvent être individuellement activées afin d'émettre un faisceau à rayons X correspondant (F) en direction du premier plan de détection (3),
- une zone (R2) pour positionner le sein (M) ;
- une unité de traitement et de commande (6), reliée aux sources de rayons X (4) pour les activer individuellement et reliée au dispositif détecteur (2) pour recevoir un signal (s1) relatif aux rayons X étant passés à travers le sein (M) et ayant été détectés par le dispositif détecteur (2), l'unité (6) étant conçue pour déduire du signal (s1) au moins une image radiographique représentative de la structure interne du sein (M) de la patiente, **caractérisé en ce qu'**au moins une première partie (PP1) des sources (4) peut se déplacer par rapport au dispositif détecteur (2) et comprenant une première source (4b) se déplaçant dans une première direction (Db) de déplacement parallèle au premier plan de détection (3) afin d'émettre des rayons X à partir d'une pluralité de positions fonctionnelles le long de ladite première direction (Db) de déplacement.

2. Appareil selon la revendication 1, dans lequel les sources (4) d'une seconde partie (PP2) de la pluralité de sources (4) sont stablement fixées par rapport au dispositif détecteur (2) et sont positionnées sur un côté (L1) et sur l'autre côté (L2) de la première source (4b) pour émettre les rayons X en direction du premier plan de détection (3) selon une pluralité d'angles différents.

3. Appareil selon les revendications 1 ou 2, dans lequel la première partie (PP1) des sources (4) comprend de plus une seconde source (4d) et une troisième source (4e) qui sont positionnées de sorte à pouvoir respectivement se déplacer sur un côté (L1) et sur l'autre côté (L2) du premier tube (4b) et à pouvoir respectivement se déplacer dans une seconde direction (Dd) et une troisième direction (De) de déplacement, lesdites directions étant placées selon un angle par rapport à la première direction (Db).

4. Appareil selon l'une quelconque des revendications précédentes, dans lequel les sources mobiles (4) sont conçues pour tourner autour d'un axis (H4, H2, H5) étant orthogonal par rapport au second plan (5) en fonction de leur position le long de la direction de déplacement respective (Dd, Db, De).

5. Appareil selon l'une quelconque des revendications précédentes, dans lequel la direction ou les directions de déplacement (Dd, Db, De) de la source ou des sources mobile(s) (4) est (sont) substantiellement parallèle(s) à un plan (T) défini par le thorax (TO) de la patiente lorsque le sein (M) est placé dans la zone de positionnement (R2).

6. Appareil selon l'une quelconque des revendications précédentes, dans lequel les sources (4) sont positionnées le long d'un arc (13).

7. Appareil selon l'une quelconque des revendications de 1 à 6, dans lequel l'appareil (1) comprend un premier cadre (12) ayant la forme d'un anneau (28) supportant les sources de rayons X (4) et le dispositif détecteur (2).

8. Appareil selon la revendication 7, **caractérisé en ce qu'**il comprend un second cadre (14) et étant aussi **caractérisé en ce que** le premier cadre (12) est soutenu de manière rotative par le second cadre (14).

9. Appareil selon la revendication 8, **caractérisé en ce qu'**il comprend des moyens (20) permettant le déplacement vertical du premier cadre (12) par rapport au second cadre (14), afin de permettre un déplacement vertical du premier cadre (12).

10. Appareil selon l'une quelconque des revendications de 5 à 7, **caractérisé en ce que** le premier cadre (12) peut pivoter autour d'un centre (A) de l'anneau (28).

11. Appareil selon l'une quelconque des revendications précédentes **caractérisé en ce qu'**il comprend une grille (7) de suppression de rayonnement diffus, la grille (7) étant interposée entre la zone de positionnement (R2) et le dispositif détecteur (2) de sorte que les rayons X qui sont passés à travers le sein (M) passent à travers cette dernière, la grille (7) de suppression de rayonnement diffus comprenant une pluralité de lamelles (26) étant positionnées à l'opposé du thorax (TO) de la patiente lorsque le sein (M) est placé dans la zone de positionnement (R2), lesdites lamelles étant orientées de sorte à converger vers la même zone (R1) à proximité d'une des sources de rayons X (4).

12. Appareil selon la revendication 11, **caractérisé en ce que** la grille (7) de suppression de rayonnement diffus comprend, dans une direction (C) orthogonale par rapport au thorax (TO) de la patiente, un nombre de lamelles (26) par centimètre étant supérieur à la moitié d'un nombre de pixels par centimètre du dispositif détecteur (2) dans cette direction (C).

13. Appareil selon les revendications 11 ou 12, **caractérisé en ce qu'**il comprend des moyens permettant de déplacer la grille (7) étant conçus pour permettre un déplacement de la grille (7) dans une direction (C) de déplacement de façon substantiellement orthogonale par rapport à une direction (K) d'extension des lamelles (26).

14. Appareil selon l'une quelconque des revendications précédentes, **caractérisé en ce qu'**il comprend des moyens (30) pour régler le spectre du faisceau de rayons X (F), associable à au moins une des sources de rayons X (4), et étant aussi **caractérisé en ce que** l'unité de traitement et de commande (6) est conçue pour commander les moyens de réglage (30), permettant le réglage du spectre du faisceau (F) émis par la source (4) entre un premier spectre d'émission et un second spectre d'émission, et pour déduire une image à partir des rayons X détectés dans le premier plan de détection (3) et relatifs au premier et au second spectre d'émission.

15. Appareil selon la revendication 14, dans lequel les moyens de réglage (30) comprennent au moins un filtre d'atténuation (34) pour le faisceau et des moyens (33) pour déplacer le filtre (34) entre une position fonctionnelle (E) dans laquelle le filtre (34) agit sur le faisceau (F) pour définir le premier spectre d'émission et une position non-fonctionnelle (D) dans laquelle le filtre (34) n'agit pas sur le faisceau (F), définissant ainsi le second spectre d'émission.
